# FASCICULE DE BREVET EUROPEEN

(11) **EP 4 099 976 B1**
(45) Date de publication et mention de la délivrance du brevet: **04.12.2024**
(21) Numéro de dépôt: 21708274.2
(22) Date de dépôt: 27.01.2021
(51) Int. Cl.: A61K 8/9789, A61Q 19/08

(54) **EXTRAIT DE MURRAYA KOENIGII ET SON UTILISATION EN COSMETIQUE**
MURRAYA-KOENIGII-EXTRAKT UND SEINE VERWENDUNG IN DER KOSMETIK
MURRAYA KOENIGII EXTRACT AND USE THEREOF IN COSMETICS

(30) Priorité: 03.02.2020 FR 2001055
(43) Date de publication de la demande: 14.12.2022
(73) Titulaire: GATTEFOSSE SAS, 69800 Saint Priest (FR)
(72) Inventeur: CHARTON, Virginie, 69740 GENAS (FR); BECHETOILLE, Nicolas, 69530 BRIGNAIS (FR); DEMARNE, Frédéric, 13008 MARSEILLE (FR)
(74) Mandataire: Cabinet Laurent & Charras
(86) Numéro de dépôt international: PCT/FR2021/050150
(87) Numéro de publication internationale: WO 2021/156556

(56) Documents cités:
- WO-A1-2014/157910
- WO-A1-2018/172436
- US-A1- 2009 169 651
- DATABASE GNPD [online] MINTEL; 21 March 2012 (2012-03-21), ANONYMOUS: "Overnight Repair Serum", XP055718305, retrieved from www.gnpd.com Database accession no. 1759348
- DATABASE GNPD [online] MINTEL; 29 January 2019 (2019-01-29), ANONYMOUS: "Night Cream", XP055718307, retrieved from www.gnpd.com Database accession no. 6293269
- VANDANA JAIN ET AL: "Murraya Koenigii: An Updated Review", INTERNATIONAL JOURNAL OF AYURVEDIC AND HERBAL MEDICINE PLOT NEAR SANPADA RAILWAY STATION, 1 January 2012 (2012-01-01), XP055291059, Retrieved from the Internet <URL:http://interscience.org.uk/issue/V2-I5/3 ijahm.pdf> [retrieved on 20210101]
- ALI GHASEMZADEH ET AL: "Optimization of ultrasound-assisted extraction of flavonoid compounds and their pharmaceutical activity from curry leaf (Murraya koenigii L.) using response surface methodology", BMC COMPLEMENTARY AND ALTERNATIVE MEDICINE, BIOMED CENTRAL LTD., LONDON, GB, vol. 14, no. 1, 28 August 2014 (2014-08-28), pages 318, XP021197070, ISSN: 1472-6882, DOI: 10.1186/1472-6882-14-318

## Description

### Domaine de l'invention

L'invention concerne un extrait de *Murraya koenigii* et une composition cosmétique comprenant ledit extrait. L'invention concerne également l'utilisation dudit extrait ou de ladite composition cosmétique pour le traitement de la peau, notamment du vieillissement cutané.

### Etat antérieur de la technique

Le derme est un tissu conjonctif fibro-élastique dense, constitué de cellules et de matrice extracellulaire (MEC) contenant, notamment, des fibres élastiques et des fibres de collagènes. Le derme a pour fonction d'assurer la nutrition et le soutien de l'épiderme ainsi que les propriétés mécaniques de la peau. Les fibroblastes, qui sont les cellules majoritaires du derme, synthétisent les protéines formant la MEC (collagènes, élastine, protéoglycanes et glycoprotéines) mais aussi les protéases (collagénases, élastases) dégradant la MEC afin de la renouveler ou de la réparer.

La genèse des fibres élastiques débute dès les premiers stades du développement et atteint un pic à la naissance. Ce processus ralentit jusqu'à la puberté et devient quasi nul à l'âge adulte. L'architecture des fibres élastiques matures est complexe et dépend du tissu, reflétant ainsi la fonction biologique du tissu considéré.

Dans la peau, les fibres élastiques se présentent sous forme de fibres individualisées, conférant ainsi à la peau son élasticité et sa résilience. Par ailleurs, les fibres élastiques ont des appellations différentes selon la proportion d'élastine présente dans ces fibres. Il est ainsi possible de différencier les fibres possédant un faible taux d'élastine et les fibres constituées de plus de 90% d'élastine ; elles sont respectivement dénommées fibres oxytalanes, fibres d'élaunine et fibres élastiques matures. Dans le derme superficiel, les fibres oxytalanes forment de fines arborisations perpendiculaires à la jonction dermo-épidermique et s'entremêlent avec les fibres d'élaunine dans le derme papillaire. Dans le derme réticulaire, les fibres élastiques sont plus épaisses et orientées parallèlement à la jonction dermo-épidermique.

Les fibres élastiques sont synthétisées au cours de l'élastogenèse qui désigne l'ensemble des processus intra- et extracellulaires conduisant à l'assemblage supramoléculaire des fibres élastiques fonctionnelles. Le coeur des fibres élastiques est composé d'élastine qui est associé à une enveloppe externe microfibrillaire principalement constituée de glycoprotéines telles que des fibrillines, fibulines, MAGPs (Microfibril-Associated Glycoprotein-1) et LTBP (LatentTGF-β-Binding Proteins). C'est l'élastine qui confère aux fibres élastiques leur élasticité et leur résilience et permet à la peau de reprendre sa position d'origine quand elle est pincée ou étirée.

L'élastine est une protéine très résistante à la protéolyse et elle fait partie des protéines les plus résistantes de l'organisme. En conditions physiologiques chez l'adulte, le renouvellement de l'élastine est quasi nul et sa demi-vie est estimée à 70 ans. Néanmoins, la synthèse d'élastine est occasionnellement réactivée pour réparer les fibres élastiques endommagées/dégradées au cours du vieillissement. Cependant, les fibres élastiques de réparation sont moins fonctionnelles que celles formées lors du développement.

Les microfibrilles constituent une matrice de nature glycoprotéique sur laquelle les monomères de tropoélastine se déposent et s'alignent avant de former des liaisons covalentes avec la fibre en cours de maturation. Les microfibrilles sont principalement constituées de fibrillines, telles que la fibrilline-1 (FBN-1) qui est fortement exprimée dans le derme. Les fibulines, telles que la fibuline-5 (FBLN-5), sont associées aux fibrillines et impliquées dans plusieurs étapes clés de l'élastogenèse telles que la liaison à la tropoélastine et l'assemblage supramoléculaire de la fibre élastique.

Le derme est affecté par le chrono-vieillissement (encore appelé vieillissement intrinsèque) qui est la conséquence d'un ensemble d'altérations génétiquement programmées. Les fibroblastes sont moins nombreux et leur activité métabolique est réduite, telle qu'une diminution de synthèse de collagène et d'élastine. Cliniquement, il en résulte l'apparition de fines rides, une perte d'élasticité progressive et une cicatrisation ralentie. Au cours du chrono-vieillissement, les fibroblastes synthétisent également plus de métalloprotéases (MMPs) qui sont impliquées dans la dégradation protéolytique des constituant de la MEC dermique. Ces enzymes peuvent alors dégrader l'élastine et les fibrillines composant les fibres élastiques. D'un point de vue histologique, on parle de phénomène d'élastolyse avec des fibres élastiques qui apparaissent fragmentées avec une diminution de leur densité en nombre et une diminution du diamètre de ces fibres.

Le vieillissement extrinsèque est la résultante de nombreux facteurs environnementaux dont la pollution, le mode de vie et l'exposition solaire (ou photo-vieillissement), et dont les effets néfastes s'ajoutent aux effets du chrono-vieillissement. Le photo-vieillissement est caractérisé par des altérations importantes au niveau du derme, ce qui conduit à la formation des rides profondément marquées et à une perte importante de l'élasticité cutanée. Les modifications observées au cours du photo-vieillissement reposent principalement sur la génération d'espèces réactives à l'oxygène (ERO), lesquelles vont engendrer une forte activation des enzymes protéolytiques provoquant une dégradation tissulaire excessive. Des protéases, telles que l'élastase neutrophile (provenant des fibroblastes et de l'infiltration des neutrophiles) et la MMP-12 (provenant des macrophages) sont sécrétées sous l'influence des UV pour dégrader la tropoélastine.

Avec l'augmentation de l'espérance de vie dans les pays développés, l'impact du vieillissement sur l'apparence et la fonctionnalité de la peau est un problème majeur qui fait l'objet de nombreuses études.

Il s'avère qu'une meilleure compréhension des mécanismes impliqués dans la physiologie du vieillissement cutané permet le développement de nouvelles stratégies capables de ralentir ou réparer ses effets indésirables.

Ainsi, des études cliniques ont montré que l'application de crèmes à base de vitamine C améliore l'aspect global de la peau et réduit les rides marquées, via l'activation de la synthèse de collagène et des fibres élastiques.

Les rétinoïdes, qui sont dérivés de la vitamine A, permettent la réduction des rides en stimulant la synthèse des fibres de collagènes et la reformation des fibres oxytalanes. De plus, ils préviennent la protéolyse excessive des protéines de la MEC en bloquant la surexpression des MMPs.

Les polyphénols, antioxydants naturellement présents dans le raisin et le vin, les thés vert et noir, les fruits et légumes, sont également de bons candidats en raison de leur propriété d'inhibition de la surexpression des MMPs.

Les extraits végétaux, qui sont riches en molécules naturelles et diverses, sont aussi de bons candidats pour stimuler des mécanismes endogènes de la photoprotection, tels que les défenses antioxydantes des cellules et l'inhibition de l'activité enzymatique des MMPs. Et plus particulièrement, le document FR 2855968 décrit des extraits d'aneth, de groseille, de cardamone, du radis noir, du petit houx, de cannelle, d'avoine, de pomme de terre et de soie pour stimuler la formation des fibres élastiques.

L'espèce végétale *Murraya koenigii* (L.) Spreng, communément appelée arbre à curry ou kaloupilé par exemple, appartient à la famille des Rutaceae. *Murraya koenigii* ou *M. koenigii* est un arbuste à feuilles caduques et aromatiques pouvant atteindre une hauteur maximale de 6 mètres. Originaire principalement de l'Inde et du Sri Lanka, on la trouve également en Asie du Sud et Sud-Est. Sa croissance est optimale dans les régions au climat tropical ou sub-tropical.

La composition phytochimique de différentes parties de *M*. *koenigii,* telles que tige foliaire (ou tige feuillue), feuille, écorce, fruit, graine, racine, est relativement décrite par l'art antérieur. Elle se caractérise principalement par la présence d'alcaloïdes de type carbazole, de composés phénoliques (flavonoïdes de type flavonols, acides phénoliques dérivés de l'acide benzoïque et de l'acide p-hydroxycinnamique, tannins), de terpénoïdes (saponines, caroténoïdes), d'huiles essentielles, de minéraux, de sucres et d'acides aminés.

*M. koenigii,* et particulièrement ses tiges foliaires et feuilles, sont utilisées traditionnellement pour l'alimentation et en médecine. Les feuilles sont très souvent utilisées pour la préparation de curry et de chutney.

Plusieurs activités liées aux applications dans le domaine cosmétique sont également rapportées à *M. koenigii* dans la littérature, à savoir anti-hyaluronidase, antimicrobienne, hydratante, anti-oxydante, dépigmentante, croissance du cheveu ou encore photoprotection.

A titre d'exemple, on peut citer le sérum de nuit de la gamme Age Smart^{®} développé par la société DERMALOGICA ou encore la crème de nuit commercialisée par la société ALDI-SÜD. De la même manière, on peut citer le document US 2009/169651 qui divulgue une composition comprenant des extraits de plantes, dont un extrait de feuilles de *M*. *koenigii,* et le document WO 2014/157910 qui décrit une composition comprenant du Thanaka obtenu par un traitement enzymatique des parties aériennes, en particulier les feuilles, de *M. koenigii*, *M. paniculata,* et *M. exotica.*

En médecine traditionnelle, on attribue aux extraits de *Murraya koenigii* des vertus pour favoriser la digestion (anti-nausée, anti-vomitif, anti-diarrhée) et lutter contre les infections (myco-)bactériennes, fongiques, virales et parasitaires. Des extraits sont décrits comme présentant des propriétés thérapeutiques et préventives pour lutter contre le stress oxydatif via la stimulation des systèmes antioxydants cellulaires. Les propriétés anti-inflammatoires sont attribuées, principalement, aux alcaloïdes contenus dans les extraits de *Murraya koenigii.* Des effets bénéfiques neurologiques (anti-anxiolytique, antidépresseur et anti-stress) et métaboliques (antidiabétique et anti-obésité) sont également soulignés chez l'animal nourrit avec des extraits de *Murraya koenigii.*

A titre d'exemple, on peut citer le document WO 2018/172436 qui divulgue une composition comprenant des actifs issus d'un broyat de feuilles séchées de *M. koenigii,* dont la metformine, pour son utilisation dans le traitement du syndrome métabolique et de troubles cognitifs.

Toutefois, il ressort de l'art antérieur que l'efficacité et les propriétés biologiques des extraits de *Murraya koenigii* sont associées à la présence d'alcaloïdes dans lesdits extraits.

En effet, comme mentionné précédemment, *Murraya koenigii* est une source importante d'alcaloïdes, notamment de type carbazole. A titre d'exemple, les carbazoles isolés à partir de l'écorce de tige sont la girinimbine, la murrayanine ou encore la mahanimbine ; à partir des tiges foliaires et feuilles les carbazoles isolés sont la mahanimbine, l'isomahanimbine, la mahanine ou encore la koenoline.

Les alcaloïdes représentent un ensemble de molécules d'origine naturelle, renfermant du carbone, de l'hydrogène et, plus spécialement, de l'azote. La plupart des alcaloïdes possèdent une action biologique, le plus souvent sur le système nerveux (psychotropes, psychoactives, stimulantes, dopantes, toniques, vomitives, calmantes, dormitives, et analgésiques). Sous forme purifiée, ces molécules dévoilent très souvent une toxicité aiguë, ainsi qu'à plus faibles doses, une activité pharmacologique apaisante, non sans effets d'accoutumance ou une toxicité chronique à long terme. Ainsi, dans une composition cosmétique, il est parfois nécessaire de diminuer la concentration en actif comprenant des alcaloïdes afin de réduire le risque de toxicité associé à ces molécules. Toutefois, cette diminution de la dose d' actif peut se faire au détriment de l'efficacité de la composition.

Il ressort de ce qui précède qu'il s'avère nécessaire de limiter, voire supprimer la présence des alcaloïdes dans les produits cosmétiques.

Les tiges foliaires (ou tiges feuillues) et les feuilles contiennent également des acides phénols dérivés de l'acide benzoïque (acides gallique, protocatéchique, syringique, ...) et de l'acide p-coumarique (acides p-coumarique, férulique, ...) mais aussi des flavonoïdes de type flavonol (quercétine). Ces molécules possèdent des propriétés biologiques utiles dans le domaine de la cosmétique, notamment antimicrobienne, antioxydante ou encore cicatrisante.

D'autre part, le choix à effectuer parmi les solvants disponibles pour l'obtention des extraits pose un certain nombre de difficultés.

Les solvants aqueux nécessitent l'ajout d'un conservateur microbiologique pour garantir leur stabilité. Or le champ d'utilisation des conservateurs est de plus en plus restreint en raison notamment des données toxicologiques requises. Le propylène glycol s'est révélé potentiellement toxique, et tend actuellement à être évité par les industriels. Le butylène glycol agrosourcé est relativement coûteux, ce qui limite son utilisation industrielle. L'éthanol, bien que présentant un bon pouvoir d'extraction seul ou en mélange dans l'eau, est particulièrement volatil et inflammable, ce qui engendre des problématiques de transport et de stockage. D'autre part, en raison de son caractère desséchant, ce solvant peut perturber le film hydrolipidique de la peau et provoquer des irritations pour les peaux sensibles.

Par ailleurs, les extraits de plantes étant souvent destinés à être formulés dans des compositions cosmétiques, il importe d'éviter les solvants d'extraction comprenant des sels ou des acides, dont on retrouvera nécessairement au moins des traces dans l'extrait. En effet, il est bien connu que la formulation, en gel ou en émulsion, d'ingrédients à base de sels au-delà d'une dose de 0,1% est particulièrement complexe. En outre, les acides, en abaissant le pH des extraits obtenus, ont pour effet de rendre la formulation en gel ou en émulsion difficile. En particulier, les produits cosmétiques destinés à l'application cutanée doivent avoir un pH adapté, de préférence proche de celui de la peau (environ pH 5,5), ou neutre. Dans ces conditions, l'utilisation de solvants contenant des acides requiert par conséquent l'ajout d'excipients, notamment des régulateurs de pH.

Le problème que se propose de résoudre l'invention est celui de trouver une alternative aux extraits végétaux existants pour lutter efficacement contre le vieillissement cutané, notamment en préservant ou améliorant l'élasticité de la peau, et qui ne présente pas les inconvénients mentionnés précédemment, notamment ceux liés à la présence des alcaloïdes dans l'extrait.

### Exposé de l'invention

Le Demandeur a constaté, de manière tout à fait surprenante, qu'un extrait de *Murraya koenigii* permet de répondre aux besoins mentionnés précédemment.

L'invention vise donc l'utilisation, de préférence non thérapeutique, d'un extrait de *Murraya koenigii* selon l'invention ou d'une composition le comprenant, pour le traitement cosmétique de la peau et/ou des muqueuses, en particulier pour l'amélioration de l'aspect de la peau et/ou des muqueuses, pour l'amélioration de la résistance et/ou de l'élasticité cutanée, pour le traitement ou la prévention du vieillissement de la peau, des rides, ou du relâchement cutané.

Selon un premier aspect, l'invention concerne un extrait de *Murraya koenigii* ou une composition cosmétique comprenant ledit extrait pour lutter contre le vieillissement cutané.

Selon un mode de réalisation particulier, l'extrait selon l'invention ou la composition cosmétique comprenant ledit extrait est utilisé pour stimuler le processus d'élastogenèse cutané.

Selon un autre mode de réalisation particulier, l'extrait selon l'invention la composition cosmétique comprenant ledit extrait est utilisé pour stimuler la formation de fibres élastiques cutanées.

Avantageusement, les fibres élastiques cutanées sont les fibres oxytalanes, les fibres d'élaunine et/ou les fibres élastiques matures.

Selon un autre mode de réalisation particulier, l'extrait selon l'invention la composition cosmétique comprenant ledit extrait est utilisé pour densifier la matrice extracellulaire cutanée (MEC).

Au sens de l'invention, par « densifier la MEC », on entend augmenter, la quantité des protéines formant la MEC (collagènes, élastine, protéoglycanes ou encore glycoprotéines).

Selon un autre mode de réalisation particulier, l'extrait selon l'invention ou la composition cosmétique comprenant ledit extrait est utilisé pour lutter contre le relâchement de la peau.

Selon un autre mode de réalisation particulier, l'extrait selon l'invention ou la composition cosmétique comprenant ledit extrait est utilisé pour cicatriser la peau.

Selon l'invention, l'extrait de *Murraya koenigii* mis en oeuvre dans les utilisations précédentes est obtenu par un procédé d'extraction solide/liquide, suivi d'une seconde étape de séparation solide/liquide et enfin d'une troisième étape de récupération de la phase liquide, caractérisée en ce que le solvant consiste en un mélange de bétaïne, de propanediol et d'eau, dans lequel les proportions molaires bétaïne:propanediol sont de 1 : strictement inférieur à 1,5 .

Le solvant peut contenir de l'eau, ou en être dépourvu. La présence d'eau dans le solvant a toutefois pour avantage de fluidifier le solvant, et ainsi de faciliter l'extraction.

Selon un mode de réalisation particulier, le solvant d'extraction mis en oeuvre pour obtenir l'extrait selon l'invention consiste en un mélange de bétaïne et de propanediol dans des proportions molaires de 1:1.

Selon un autre mode de réalisation particulier, le solvant d'extraction mis en oeuvre pour obtenir l'extrait selon l'invention consiste en un mélange de bétaïne, de propanediol et d'eau dans des proportions molaires de 1: strictement inférieur à 1,5 et l'eau représente entre 15 et 35% en poids du solvant.

Selon un autre mode de réalisation particulier, le solvant d'extraction mis en oeuvre pour obtenir l'extrait selon l'invention consiste en un mélange de bétaïne, de propanediol et d'eau dans des proportions molaires de 1:1 et l'eau représente avantageusement entre 15 et 35% en poids du solvant.

Avantageusement, le solvant d'extraction mis en oeuvre pour obtenir l'extrait selon l'invention consiste en un mélange de bétaïne, de propanediol et d'eau dans des proportions molaires 1:1:5.

Selon un mode de réalisation particulier, toute ou partie de la plante *Murraya koenigii* peut être utilisée comme extrait pour lutter contre le vieillissement cutané. En pratique, la partie de la plante utilisée comme extrait est choisie dans le groupe comprenant le fruit, la fleur, la graine, la racine, la feuille, la tige, la tige foliaire (ou tige feuillue) et la jeune pousse.

Avantageusement, la partie de la plante mise en oeuvre est la tige foliaire (ou tige feuillue) et/ou la feuille.

Selon un mode de réalisation particulier, la partie de la plante mise en oeuvre est fraîche, congelée, séchée, entière, coupée et/ou broyée.

Avantageusement, il s'agit de la tige foliaire et/ou la feuille séchées, de préférence de la tige foliaire et/ou la feuille séchées et broyées.

De préférence, il s'agit de la tige foliaire séchée, de préférence de la tige foliaire séchée et broyée.

Selon un autre aspect, l'invention concerne un extrait de *Murraya koenigii* obtenu par un procédé d'extraction solide/liquide, suivi d'une seconde étape de séparation solide/liquide et enfin d'une troisième étape de récupération de la phase liquide, caractérisée en ce que le solvant consiste en un mélange de bétaïne, de propanediol et d'eau.

Selon un mode de réalisation particulier, le solvant d'extraction mis en oeuvre pour obtenir l'extrait selon l'invention consiste en un mélange de bétaïne, de propanediol et d'eau dans des proportions molaires 1:1:5.

Selon l'invention, toute ou partie de la plante *Murraya koenigii* peut être mise en oeuvre dans l'invention. En pratique, la partie de la plante mise en oeuvre dans le procédé d'extraction est choisie dans le groupe comprenant la tige, la feuille, le fruit, la fleur, la graine, la racine, la tige foliaire (ou tige feuillue) et la jeune pousse.

De manière particulièrement préférée, la partie de la plante mise en oeuvre est la tige foliaire et/ou la feuille. Il peut s'agir d'une tige foliaire et/ou d'une feuille fraiches, congelées, sèches, entières, coupées ou broyées.

Avantageusement, il s'agit de la tige foliaire et/ou la feuille séchées, de préférence de la tige foliaire et/ou la feuille séchées et broyées.

De préférence, il s'agit de la tige foliaire séchée, de préférence de la tige foliaire séchée et broyée.

Selon un mode de réalisation particulier, l'extrait de *Murraya koenigii* selon l'invention est dépourvu d'alcaloïdes, avantageusement de carbazoles, de préférence de mahanimbine, de murrayanine de girinimbine, d'isomahanimbine, de la mahanine et/ou de koenoline.

Au sens de l'invention, par « dépourvu en alcaloïdes », on entend une absence d'alcaloïdes dans l'extrait ou une présence d'alcaloïdes à l'état de trace à une concentration inférieure à 5 mg/100g d'extrait.

Selon un mode de réalisation particulier, l'extrait de *Murraya koenigii* selon l'invention est riche en acides phénoliques, de préférence en dérivés de l'acide p-hydroxycinnamique, et/ou en flavonoïdes, de préférence de type flavonols.

Au sens de l'invention, par « riche » on désigne :
- une concentration d'acides phénoliques supérieure à 100mg/kg d'extrait, avantageusement supérieure à 250 mg/kg d'extrait ; et
- une concentration de flavonoïdes supérieure à 50 mg/kg d'extrait, avantageusement supérieure à 100 mg/kg d'extrait.

Le solvant selon l'invention est notamment liquide à température ambiante, ce qui facilite la mise en oeuvre du procédé conduisant à l'extrait de l'invention.

En pratique, le solvant utilisé peut être produit en mélangeant la bétaïne et le propanediol ou la bétaïne, le propanediol et l'eau dans un réacteur agité, jusqu'à l'obtention d'un mélange limpide incolore. Ce mélange peut être réalisé à une température comprise entre 2°C et 100°C et pendant 30 minutes à 6 heures, préférentiellement 40°C à 70°C pendant 1 heure à 2 heures.

L'extrait selon l'invention est obtenu par un procédé mettant en oeuvre une étape d'extraction solide/liquide.

L'extraction solide/liquide peut être effectuée par différentes techniques bien connues de l'homme du métier, telles que macération, remacération, digestion, macération dynamique, décoction, extraction en lit fluide, extraction assistée par micro-ondes, extraction assistée par ultra-sons, extraction à contre-courant, percolation, re-percolation, lixiviation, extraction sous pression réduite, diacolation.

En pratique le rapport massique plante/solvant appliqué pour l'étape d'extraction est compris entre 1:99 et 50:50, avantageusement entre 3:97 et 10:90. De préférence, il s'agit d'un rapport massique de tiges foliaires (ou tiges feuillues) et/ou de feuilles de *Murraya koenigii* séchées broyées/solvant compris entre 1:99 et 50:50, avantageusement entre 3:97 et 10:90. L'étape d'extraction est effectuée de préférence à une température comprise entre 2 et 100°C, plus préférentiellement entre 20 et 80°C. L'étape d'extraction peut être maintenue pendant quelques minutes à plusieurs jours.

De manière à optimiser l'extraction des composés actifs tout en protégeant ces composés de l'oxydation par l'oxygène de l'air, l'étape d'extraction solide/liquide est avantageusement réalisée sous agitation et/ou sous atmosphère d'azote.

Selon l'invention, l'étape d'extraction solide/liquide est suivie d'une étape de séparation solide/liquide, l'objectif étant de récupérer la phase liquide, aussi appelée filtrat de séparation solide/liquide, contenant la matière active. Cette séparation peut être effectuée par toute technique connue de l'homme du métier, en particulier l'égouttage, le pressage, l'essorage, la centrifugation ou la filtration.

De manière optionnelle, l'étape de séparation liquide/solide peut être suivie d'une étape de concentration, laquelle permet d'obtenir un concentré, sous forme liquide ou semi-solide selon le facteur de concentration. En pratique, l'étape de concentration peut être effectuée par évaporation sous pression réduite ou osmose inverse.

De préférence, le filtrat de séparation solide/liquide ou le concentré subissent en outre une ou plusieurs étapes de clarification. Pour réaliser cette étape de clarification, l'homme du métier pourra utiliser tout type de filtration frontale et/ou tangentielle connue dans le domaine considéré.

Selon l'invention, de manière optionnelle, l'étape de séparation liquide/solide ou de concentration ou de clarification peut être suivie d'une étape de fractionnement, laquelle permet d'obtenir une fraction d'une ou plusieurs familles phytochimiques, sous forme liquide ou semi-solide selon le facteur de concentration. En pratique, l'étape de fractionnement peut être effectuée par toute technique connue de l'homme du métier, en particulier par chromatographie sous pression réduite, ultrafiltration ou nanofiltration.

Enfin, en vue d'un conditionnement, le procédé permettant l'obtention de l'extrait liquide selon l'invention peut comprendre une filtration stérilisante. La filtration stérilisante est classiquement réalisée en filtrant le produit à travers un filtre comprenant des pores d'un diamètre de 0,22 µm. De préférence, l'étape de filtration stérilisante est l'étape finale du procédé.

Le Demandeur a en outre découvert que l'extrait de *Murraya koenigii* selon l'invention possède des propriétés biologiques particulièrement intéressantes, en particulier sur la stimulation de la synthèse de protéines de la peau.

L'extrait de *Murraya koenigii* selon l'invention est capable de moduler l'expression de protéines associées à l'élasticité cutanée.

En effet et tel que détaillé dans la partie expérimentale, l'extrait de *Murraya koenigii* selon l'invention a notamment pour propriété de stimuler l'expression de protéines appartenant aux fibres élastiques, notamment l'élastine, la fibrilline-1, la fibrilline-2 ou encore la fibuline-5. L'extrait selon l'invention est donc particulièrement intéressant pour des utilisations cosmétiques, en particulier visant l'amélioration de l'aspect de la peau, ou encore la préservation et/ou l'amélioration de l'élasticité de la peau. En conséquence, l'extrait selon l'invention permet de lutter contre les effets du vieillissement cutané, de préférence le vieillissement intrinsèque et/ou le vieillissement extrinsèque, en particulier le photo-vieillissement.

L'extrait selon l'invention a comme avantages de :
- présenter des propriétés biologiques (pro-élastogéniques) en absence/avec une faible teneur d'alcaloïdes ;
- d'être totalement naturel, c'est-à-dire que tous les composants sont naturels ; et
- d'être compatible et parfaitement formulable dans tous les produits de soin cosmétique.

Selon un autre aspect, l'invention concerne une composition comprenant l'extrait de l'invention, de préférence une composition cosmétique, c'est-à-dire adaptée à l'application topique sur la peau et/ou les muqueuses, et/ou les phanères.

Selon un mode de réalisation particulier, l'extrait selon l'invention représente entre 0,1% et 10% en poids de la composition, avantageusement entre 0,5% et 5%.

Selon un mode de réalisation particulier, la composition selon l'invention est une composition de soin de la peau, une composition de soin anti-âge ou une composition de protection solaire.

La composition selon l'invention peut se présenter sous toutes les formes galéniques normalement utilisées pour une application topique sur la peau et/ou les muqueuses, et/ou les phanères, par exemple sous forme anhydre, sous forme d'une émulsion huile-dans-eau, d'une émulsion eau-dans-huile, d'une émulsion multiple, d'une émulsion siliconée, d'une microémulsion, d'une nanoémulsion, d'un gel, d'une solution aqueuse ou d'une solution hydro-alcoolique.

Cette composition peut être plus ou moins fluide et se présenter, par exemple, sous forme d'une crème, d'une pommade, d'un lait, d'une lotion, d'un sérum, ou d'un gel.

La composition cosmétique peut contenir les excipients habituellement utilisés dans les domaines cosmétique, tels que les matières grasses, les tensioactifs détergents et/ou conditionnants, les émulsionnants et co-émulsionnants, les gélifiants hydrophiles ou lipophiles, les conservateurs, les antioxydants, les solvants, les agents exfoliants, les parfums, les charges, les filtres hydrophiles et lipophiles, les matières colorantes, les neutralisants, les agents pro-pénétrants, et les polymères. Ces types d'excipients sont tous bien connus de l'Homme du métier.

En pratique, les quantités de ces différents excipients sont celles classiquement utilisées dans les domaines considérés, et la somme des excipients représente de préférence 0,01% à 30% du poids total de la composition.

Comme matières grasses appropriées, on peut citer les huiles minérales, les huiles d'origine animale (telle la lanoline), les huiles végétales, les huiles de synthèse (comme par exemple l'isopropyl le myristate, l'octyldodecyl, l'isostearyl isostearate, le decyl oleate, l'isopropyl le palmitate), les huiles siliconées (la cyclomethicone, la dimethicone) et les huiles fluorées. On peut utiliser comme matières grasses des alcools gras, des acides gras, des cires et des gommes, et en particulier des élastomères de silicone.

Comme tensioactifs détergents et/ou conditionnants appropriés, on peut citer les tensioactifs non ioniques, anioniques, cationiques ou amphotères, et leurs mélanges, tels que par exemple les alkylsulfates, les alkyléthersulfates tels que le lauryl éther sulfate de sodium, les alkyl bétaïnes telles que la cocamidopropylbetaïne, ou les sels d'ammonium quaternaires.

Comme émulsionnants et co-émulsionnants appropriés, on peut citer par exemple les esters de polyglycérols et d'acide gras, les esters de sucrose et d'acide gras, les esters de sorbitane et d'acide gras, les esters d'acide gras et de sorbitane oxyéthylénés, les ethers d'alcool gras et de PEG, les esters de glycérol et d'acide gras, les alkyl sulfates, les alkyl ether sulfates, les alkyl phosphates, les alkyl polyglucosides, les dimethicone copolyols.

Comme gélifiants hydrophiles appropriés, on peut citer par exemple les polymères carboxyvinyliques (carbomers), les copolymères acryliques tels que les copolymères d'acrylates/alkylacrylates, les polyacrylamides, les polysaccharides tels que la gomme xanthane, la gomme guar, les gommes naturelles telles que la gomme de cellulose et dérivés, les amidons et leurs dérivés, les argiles et les copolymères d'acide 2-acrylamido-2-méthylpropane.

Comme gélifiants lipophiles appropriés, on peut citer par exemple les argiles modifiées comme les bentones, les sels métalliques d'acides gras, la silice hydrophobe et l'éthylcellulose.

Comme conservateurs appropriés, on peut citer par exemple les acides benzoïque, sorbique, propionique, salicylique, dehydroacétique et leurs sels, l'alcool benzylique, l'éthylhexylglycérine, les parabens, leurs sels et esters, le triclosan, l'imidazolidinyl urée, le 5 phenoxyethanol, la DMDM hydantoïne, le diazolidinyl urée, la chlorphenesine.

Comme antioxydants appropriés, on peut citer par exemple les agents chélatants tels que l'EDTA et ses sels, le metabisulfite de sodium, les acides salicylique, ascorbique et citrique et leurs sels, le tartrate de sodium, le gluconate de sodium, les caroténoïdes et les tocophérols.

Comme solvants utilisables dans la composition cosmétique (distinct du solvant d'extraction), on peut citer l'eau, la glycérine, le propylène glycol ou encore le sorbitol.

Comme agents exfoliants appropriés, on peut citer par exemple les exfoliants chimiques tels que les AHA, et les exfoliants physiques tels que les poudres naturelles ou synthétiques.

Comme charges appropriées, on peut citer par exemple le talc, le kaolin, le mica, la séricite, le magnésium carbonate, l'aluminium silicate, le magnésium silicate, les poudres organiques telles que le nylon.

Comme colorants appropriés, on peut citer par exemple les colorants lipophiles, les colorants hydrophiles, les pigments et les nacres habituellement utilisés dans les compositions cosmétiques et leurs mélanges.

Comme neutralisants appropriés, on peut citer par exemple la soude, la triéthanolamine, l'aminométhyl propanol ou encore l'hydroxyde de potassium.

Comme agents pro-pénétrants appropriés, on peut citer par exemple les alcools et glycols (éthanol, propylène glycol), l'éthoxydiglycol, les alcools et acides gras (acide oléique), les esters d'acides gras ou encore le diméthyl isosorbide.

La composition de l'invention peut également contenir en outre d'autres actifs que l'extrait selon l'invention. Comme actifs appropriés, on peut citer par exemple les anti-radicalaires ou plus généralement les antioxydants, les blanchissants, les pigmentants, les émollients, les hydratants, les anti-séborrhéiques, les anti-inflammatoires, les anti-acnéiques, les agents kératolytiques et/ou desquamants, les agents antirides et tenseurs, les agents drainants, les agents anti-irritants, les agents apaisants, les vitamines et leurs mélanges, les agents matifiants, les actifs anti-âge tel que le rétinol, les cicatrisants, les antiseptiques et les huiles essentielles.

L'invention et les avantages qui en découlent ressortiront bien des exemples de réalisation suivants.

### Brève description des figures

La manière dont l'invention peut être réalisée et les avantages qui en découlent ressortiront mieux des exemples de réalisation qui suivent, donnés à titre indicatif et non limitatif, à l'appui des figures annexées.

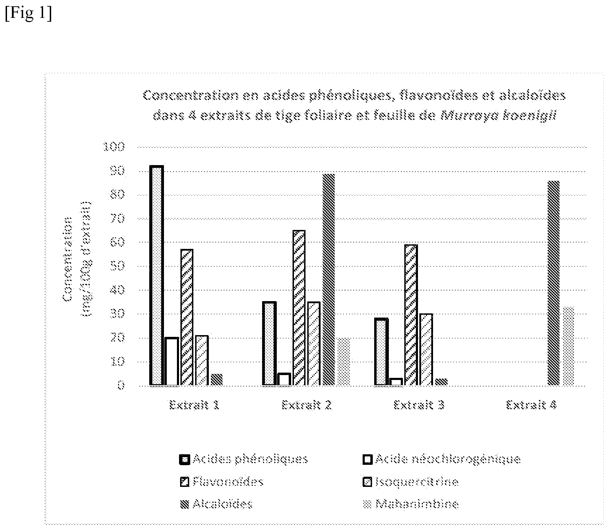

[Fig 1] La figure 1 représente la teneur en acides phénoliques, flavonoïdes et alcaloïdes de 4 extraits de *Murraya koenigii.*

### Description détaillée de l'invention

### 1/ Préparation des extraits de Murraya koenigii

Extrait 1 : Extrait de *Murraya koenigii* obtenu à partir de tiges foliaires et feuilles séchées et broyées par extraction avec un solvant selon l'invention consistant en un mélange de bétaïne, de propanediol et d'eau dans un ratio molaire 1:1:5 (BPE115) sous agitation continue pendant 3h à 70°C de la plante et du solvant selon le ratio massique 8:92. Après extraction, le résidu végétal est éliminé par un procédé mécanique (essorage par exemple) et l'extrait brut est filtré jusqu'à 0,22µm.

Extrait 1bis : Extrait obtenu selon les mêmes conditions opératoires que celles décrites pour l'extrait 1 précédemment, à l'exception du ratio massique plante/solvant qui est de 5:95. Extrait 2 : Extrait de *Murraya koenigii* obtenu à partir de tiges foliaires et feuilles séchées et broyées par extraction avec un solvant éthanol/eau dans un ratio massique 70:30 sous agitation continue pendant 3h à 70°C de la plante et du solvant selon le ratio massique 8:92. Après extraction, le résidu végétal est éliminé par un procédé mécanique (essorage par exemple). L'extrait brut est décoloré sur charbon actif charbon Acticarbone CPW CECA à 0,08% puis l'éthanol est évaporé sous pression réduite et l'extrait concentré aqueux obtenu solubilisé dans du propanediol tel que l'extrait sec final est égal à l'extrait sec de l'extrait hydro-éthanolique.

Extrait 3 : Fraction de l'extrait hydro-éthanolique décoloré décrit pour l'extrait 2 enrichie en polyphénols et contenant seulement des traces d'alcaloïdes (< à 10mg/100g de fraction). La fraction est obtenue par filtration sur résine Amberlite FPX 68 en colonne de l'extrait éthanol/eau 70:30 décoloré sur charbon actif, tel que décrit pour l'extrait 2, le filtrat non adsorbé constitue l'extrait 3.

Extrait 4 : Fraction de l'extrait hydro-éthanolique décoloré décrit pour l'extrait 2 enrichie en alcaloïdes et dépourvue de polyphénols. La fraction est obtenue par filtration/adsorption sur résine Amberlite FPX 68 en colonne de l'extrait éthanol/eau 70:30 décoloré sur charbon actif décrit pour l'extrait 2, puis désorption avec de l'éthanol des molécules adsorbées sur la résine.

### 2/ Caractérisation du pouvoir d'extraction de Murraya koenigii par le solvant bétaïne/propanediol/eau dans des proportions molaires différentes

Le pouvoir d'extraction des polyphénols et des alcaloïdes des tiges foliaires (ou tiges feuillues) et feuilles de *Murraya koenigii* a été étudié pour 6 compositions de mélanges bétaïne/propanediol/eau (dans des rapports molaires différents), représentées dans le tableau 1.

**[Tableau 1]**

| **BPE ratio molaire** | **1:1:4** | **1:1:5** | **1:1,5:3** | **1:1,5:5** | **1:2:3** | **1:2:6** |
|---|---|---|---|---|---|---|
| **Bétaïne (g)** | 44,16 | 41,35 | 41,06 | 36,45 | 36,23 | 31,04 |
| **Propanediol (g)** | 28,68 | 26,86 | 40,00 | 35,52 | 47,06 | 40,32 |
| **Eau (g)** | 27,16 | 31,79 | 18,94 | 28,03 | 16,71 | 28,64 |

Chaque solvant est préparé par mélange de bétaïne, de propanediol et d'eau à 70°C pendant environ 1h sous agitation mécanique. Chaque extrait est obtenu par extraction à 70°C pendant 4h de 5% de tiges foliaires et feuilles sèches broyées de *Murraya koenigii* et 95% de solvant. Après extraction, le résidu végétal est éliminé par un procédé mécanique et l'extrait brut est filtré jusqu'au seuil de filtration stérilisante (0,22 µm).

Les flavonoïdes et les acides phénoliques (appartenant au groupe des polyphénols) ont été analysés par RP-UHPLC-UV, les acides phénoliques ont été repérés et quantifiés par rapport à la droite d'étalonnage de l'acide chlorogénique à 287nm et les flavonoïdes ont été repérés et quantifiés par rapport à la droite d'étalonnage de l'isoquercitrine à 355nm. L'indice Gardner de chaque extrait a été mesuré après dilution au 1/2 m/v dans l'eau avec un colorimètre Lico 100.

La stabilité physique (aspect visuel) des 6 extraits conditionnés en flacon verre a été observée après 7 jours de stockage à 3 températures différentes (4, 25 et 40°C).

Les concentrations en flavonoïdes et en acides phénoliques pour chaque extrait sont représentées dans le tableau 2.

**[Tableau 2]**

| | **Concentration (mg/100g d'extrait)** | | **Indice de Gardner** |
|---|---|---|---|
| **Solvant** | **Flavonoïdes** | **Acides phénoliques** | |
| **BPE 1:1:4** | 11 | 24 | 10 |
| **BPE 1:1:5** | 12 | 27 | 9 |
| **BPE 1:1,5:3** | 10 | 21 | 8 |
| **BPE 1:1,5:5** | 11 | 24 | 10 |
| **BPE 1:2:3** | 11 | 20 | 7 |
| **BPE 1:2:6** | 12 | 25 | 11 |

Les 6 solvants présentent une performance d'extraction des flavonoïdes similaire, caractérisée par une concentration comprise entre 10 et 12 mg/100 g d'extrait. En ce qui concerne leur performance d'extraction vis-à-vis de acides phénoliques, celle-ci est comprise entre 20 et 27 mg/100 g d'extrait.

Tous les solvants testés dans cet exemple sont efficaces pour obtenir un extrait de *Murraya koenigii* comprenant une quantité efficace de flavonoïdes et d'acides phénoliques pour être utilisé en cosmétique. Le solvant le plus performant pour l'extraction de flavonoïdes et d'acides phénoliques est le solvant consistant en un mélange de bétaïne, de propanediol et d'eau dans des proportions molaires 1:1:5.

### 3/ Comparaison de la teneur en acides phénoliques, flavonoïdes et alcaloïdes de l'extrait de Murraya koenigii selon l'invention par rapport à d'autres extraits

Les teneurs en alcaloïdes, acides phénoliques et flavonoïdes des 4 extraits de *Murraya koenigii* décrits à l'exemple 1 ont été évaluées par RP-HPLC-UV. Les acides phénoliques ont été repérés et quantifiés par rapport à la droite d'étalonnage de l'acide néochlorogénique à 287nm, les flavonoïdes ont été repérés et quantifiés par rapport à la droite d'étalonnage de l'isoquercitrine à 355nm et les alcaloïdes ont été repérés et quantifiés par rapport à la droite d'étalonnage de la mahanimbine à 287nm.

Les résultats obtenus sont illustrés par la figure 1.

La composition des 4 extraits est nettement distincte d'un point de vue qualitatif et quantitatif concernant les 3 familles de métabolites secondaires étudiées. Ainsi :
- L'extrait 1 (BPE115 selon l'invention) est caractérisé par la concentration en acides phénoliques la plus élevée, une concentration en flavonoïdes équivalente à celle des extraits 2 et 3, et la présence de traces d'alcaloïdes ;
- L'extrait 2 se caractérise par une concentration significative pour chacune des 3 familles de composés (acides phénoliques, flavonoïdes et alcaloïdes) ;
- L'extrait 3 est caractérisé par une concentration similaire en polyphénols à celle de l'extrait 2, mais ne contient que des traces d'alcaloïdes ; et
- L'extrait 4 est caractérisé par une concentration en alcaloïdes similaire à celle de l'extrait 2, mais ne contient pas de polyphénols.

Il ressort de ce qui précède que seul le solvant selon l'invention consistant en un mélange de bétaïne, de propanediol et d'eau dans de proportions molaires de 1:1:5 permet d'obtenir un extrait de *Murraya koenigii* riches en flavonoïdes et en acides phénoliques et présentant une teneur en alcaloïdes très faible, à l'état de traces.

### 4/ Comparaison de la teneur en acides phénoliques, flavonoïdes et alcaloïdes de l'extrait de Murraya koenigii selon l'invention par rapport au ratio plante/solvant

Les teneurs en alcaloïdes, acides phénoliques et flavonoïdes des extraits 1 et 1bis de *Murraya koenigii* décrits à l'exemple 1 ont été évaluées par RP-HPLC-UV selon les conditions décrites dans l'exemple 3.

Les résultats sont décrits dans le tableau 3 ci-dessous.

**[Tableau 3]**

| | | **Concentration (mg/100g d'extrait)** | | |
|---|---|---|---|---|
| **Extrait** | **Ratio plante/solvant** | **Acides phénoliques** | **Flavonoïdes** | **Alcaloïdes** |
| **Extrait 1bis** | 5:95 | 45 | 26 | traces |
| **Extrait 1** | 8:92 | 92 | 57 | 5 |

Il ressort de ce qui précède que la réduction du ratio plante/solvant à 5:95 (extrait 1bis), par rapport au ratio 8:92 (Extrait 1), permet d'obtenir un extrait ne contenant que des traces non quantifiables d'alcaloïdes tout en présentant une concentration en acides phénoliques et flavonoïdes intéressante.

### 5/ Etude de l'effet de l'extrait de Murraya koenigii selon l'invention (extrait 1) sur l'expression de protéines associées à la formation des fibres élastiques

### 5.1/ Méthode

Le contenu protéique (protéome) et ses variations d'expression dans des fibroblastes dermiques humains normaux traités ou non avec les 4 extraits de *Murraya koenigii* décrits à l'exemple 1 (extraits 1, 2, 3 et 4) ont été identifiés par la nano-méthode de chromatographie liquide couplée à la spectrométrie en masse tandem (nLC-MS/MS). Cette méthode analytique est quantitative, sensible et reproductible et permet l'identification des protéines exprimées dans les cellules par une analyse bio-informatique (CORAVALID).

### 5.2/ Protocole

L'étude protéomique consiste à analyser qualitativement et quantitativement les protéines exprimées dans des cellules non traitées (NT) par rapport à des cellules traitées par les 4 extraits de feuilles de *Murraya koenigii* décrits à l'exemple 1. Les 4 extraits sont testés à leur concentration maximale non cytotoxique, à savoir l'extrait 1 (invention) à 0,2% ; l'extrait 2 (propanediol/eau) à 0,04% ; l'extrait 3 (éthanol/eau) à 1% et l'extrait 4 (éthanol) à 0,1%, et incubés dans le milieu des cultures cellulaires pendant 24h.

Les fibroblastes sont ensuite lavés avec un tampon phosphate salin à froid, puis récupérés et congelés à -80°C. Les lysats protéiques et les extraits peptidiques correspondants sont obtenus selon la méthode décrite par Wiśniewski JR *et al.,* Nat Methods. 2009; 6(5):359-62.

250 ng de peptides sont injectés dans une pré-colonne C18 (300 µm × 5 mm ; diamètre des particules 5 µm ; Thermo Scientific) puis élués dans une colonne C18 (75 µm × 500 mm ; diamètre des particules 2µm ; Thermo Scientific). La nano-chromatographie liquide est effectuée avec l'équipement Ultimate 3000 (Dionex) en appliquant un gradient d'acétonitrile de 60 à 35% pendant 60 minutes à un débit de 300 nl/min. Les données sont générées avec le spectromètre de masse Q-Exactive (Thermo Scientific).

Le protéome des cellules est analysé à l'aide de l'algorithme SEQUEST-HT sur la base de données UNIPROT. La quantification des protéines est réalisée par l'algorithme Minora et le ratio cellules traitées avec un extrait de *Murraya koenigii*/cellules non traitées est calculé selon la méthode Pairwise Ratio Based puis exprimé comme la médiane géométrique de toutes les données générées.

L'étude est basée sur 3 expériences indépendantes (n=3). Le test d'hypothèse réalisé est une analyse de la variance (ANOVA) pour comparer le protéome des fibroblastes non traités par rapport aux fibroblastes traités avec les 4 extraits de *Murraya koenigii* décrits à l'exemple 1.

### 5.3/ Résultats

L'étude a permis d'identifier 4 protéines associées aux fibres élastiques et dont l'expression est significativement augmentée dans les fibroblastes dermiques traités avec l'extrait bétaïne/propanediol/eau (ratio molaire 1: 1:5) de *Murraya koenigii.*

Les résultats sont présentés dans le tableau 4 et sont exprimées en ratio d'expression cellules traitées avec un extrait de *Murraya koenigii*/cellules non traitées et une moyenne est calculée sur les 3 expériences.

**[Tableau 4]**

| **Protéines** | **Ratio Extrait linon traité** | **Valeur p** |
|---|---|---|
| **Elastine** | 2,18 | 6,6E-14 |
| **Fibrilline-1** | 2,18 | 1,0E-17 |
| **Fibrilline-2** | 2,07 | 2,8E-12 |
| **Fibuline-5** | 1,69 | 9,4E-11 |

De manière surprenante, seul l'extrait de *Murraya koenigii* selon l'invention (extrait 1: BPE115) augmente significativement l'expression protéique de l'élastine et de ses 3 partenaires, fibrilline-1, fibrilline-2 et fibuline-5 dans les fibroblastes dermiques humains normaux. Les 3 autres extraits (extrait 2 : propanediol/eau ; extrait 3 : éthanol/eau et extrait 4 : éthanol) n'ont pas d'effet sur les protéines des fibres élastiques (données non présentées).

### 5.4/ Conclusion

L'extrait de *Murraya koenigii* selon l'invention, à savoir obtenu au moyen du solvant consistant en un mélange bétaïne/propanediol/eau (ratio molaire 1: 1:5 ; extrait 1) augmente l'expression de protéines appartenant aux fibres élastiques. Les fibroblastes dermiques humains normaux traités avec l'extrait bétaïne/propanediol/eau de *Murraya koenigii* selon l'invention présentent donc un phénotype favorable pour la régénération et le renouvellement des fibres élastiques de la peau.

### 6/ Etude de l'effet des extraits de Murraya koenigii (extrait 1 et 1bis) sur la synthèse d'élastine, de fibrilline-1 et de fibuline-5 dans des culture 2D de fibroblastes humains normaux

### 6.1/ Méthode

Cette étude utilise l'immuno-marquage fluorescent *in situ* couplée à une analyse d'image pour mesurer les synthèses d'élastine, de fibrilline-1 et de fibuline-5 déposées dans des cultures en monocouche de fibroblastes dermiques traités ou non avec les extraits 1 et 1bis selon l'invention (bétaïne/propanediol/eau, ratio molaire 1:1:5) de *Murraya koenigii.*

### 6.2/ Protocole

Des fibroblastes dermiques humains normaux sont traités pendant 48h avec les extraits selon l'invention (bétaïne/propanediol/eau et ratio molaire 1:1:5) de *Murraya koenigii* obtenu à l'exemple 1 (extraits 1 et 1bis), à la concentration finale de 0,2%. Les cellules non traitées (NT) sont utilisées comme contrôle. Les cellules sont ensuite lavées avec un tampon phosphate salin avant d'être fixées et saturées à la BSA (albumine sérique bovine) à 1% pendant 30 minutes. Les cellules sont incubées avec l'anticorps primaire (anti-élastine ; anti-fibrilline-1 ou anti-fibuline-5) pendant 1 heure, lavées en tampon PBS, puis incubées avec l'anticorps secondaire lié au fluorochrome Alexa 594 pendant 1 heure. La fluorescence est lue sur un spectrophotomètre-imageur Cytation 5 (Biotek) avec les filtres adéquates. Les mesures de fluorescence par analyse d'image sont effectuées sur des paramètres communs d'acquisition.

L'étude est basée sur 3 expériences indépendantes (n=3). Le test statistique est le 2 Way-ANOVA suivi par des comparaisons multiples de Bonferroni pour comparer la synthèse des protéines d'intérêt déposées dans les cultures de fibroblastes dermiques non traités par rapport aux fibroblastes traités avec les extraits 1 et 1bis (bétaïne/propanediol/eau, ratio molaire 1:1:5) de *Murraya koenigii* selon l'invention.

### 6.3/ Résultats

### 6.3.1/ Synthèse d'élastine

Nous avons quantifié la synthèse d'élastine déposée dans les fibroblastes dermiques traités ou non avec les extraits 1 et 1bis (bétaïne/propanediol/eau, ratio molaire 1:1:5) de *Murraya koenigii.* Les résultats sont présentés dans le tableau 5 et exprimés en % par rapport aux cellules non traitées (NT). Les valeurs sont exprimées en moyenne ± écart type sur les 3 expériences (n=3).

**[Tableau 5]**

| | **Non traité** | **Extrait 1** | **Extrait 1bis** |
|---|---|---|---|
| Exp_1 | 100 ± 34 | 158 ± 38 | 150 ± 22 |
| Exp_2 | 100 ± 21 | 151 ± 26 | 168 ± 27 |
| Exp_3 | 100 ± 23 | 178 ± 21 | 172 ± 20 |
| **n=3** | | **62% ± 14** | **63% ± 12** |
| Statistiques: ****: p<0,0001 | | **** | **** |

Les extraits 1 et 1 bis de *Murraya koenigii* obtenus selon l'invention (bétaïne/propanediol/eau, ratio molaire 1:1:5) induisent de manière équivalente une augmentation significative de la synthèse protéique d'élastine déposée (+62% et +63%, respectivement) dans les fibroblastes dermiques.

Le solvant d'extraction seul (bétaïne/propanediol/eau ratio molaire 1:1:5 sans *Murraya koenigii*) n'induit pas d'augmentation de synthèse d'élastine déposée (données non présentées).

### 6.3.2/ Synthèse de fibrilline-1

Nous avons quantifié la synthèse de fibrilline-1 déposée dans les fibroblastes dermiques traités ou non avec les extraits 1 et 1bis (bétaïne/propanediol/eau, ratio molaire 1:1:5) de *Murraya koenigii.* Les résultats sont présentés dans le tableau 6 et exprimés en % par rapport aux cellules non traitées (NT). Les valeurs sont exprimées en moyenne ± écart type sur les 3 expériences (n=3).

**[Tableau 6]**

| | **Non traité** | **Extrait 1** | **Extrait 1bis** |
|---|---|---|---|
| Exp_1 | 100 ± 11 | 189 ± 16 | 152 ± 30 |
| Exp_2 | 100 ± 32 | 129 ± 44 | 137 ± 26 |
| Exp_3 | 100 ± 43 | 140 ± 38 | 128 ± 33 |
| **n=3** | | **53% ± 32** | **39% ± 12** |
| Statistiques: ****: p<0,0001 | | **** | ** |

Les extraits 1 et 1 bis de *Murraya koenigii* obtenus selon l'invention (bétaïne/propanediol/eau, ratio molaire 1:1:5) induisent une augmentation significative de la synthèse protéique de fibrilline-1 déposée dans les fibroblastes dermiques. Les extraits 1 et 1bis ne sont statistiquement pas différents entre eux même si l'extrait 1 augmente (+53%) plus fortement que l'extrait 1bis (+39%) la synthèse protéique de fibrilline-1 dans les fibroblastes dermiques.

Le solvant d'extraction seul (bétaïne/propanediol/eau ratio molaire 1:1:5 *sans Murraya koenigii*) n'induit pas d'augmentation de synthèse de fibrilline-1 déposée (données non présentées).

### 6.3.3/ Synthèse de fibuline-5

Nous avons quantifié la synthèse de fibuline-5 déposée dans les fibroblastes dermiques traités ou non avec les extraits 1 et 1bis (bétaïne/propanediol/eau, ratio molaire 1:1:5) de *Murraya koenigii.* Les résultats sont présentés dans le tableau 7 et exprimés en % par rapport aux cellules non traitées (NT). Les valeurs sont exprimées en moyenne ± écart type sur les 3 expériences (n=3).

**[Tableau 7]**

| | **Non traité** | **Extrait 1** | **Extrait 1bis** |
|---|---|---|---|
| Exp_1 | 100 ± 53 | 260 ± 121 | 162 ± 56 |
| Exp_2 | 100 ± 56 | 222 ± 133 | 196 + 74 |
| Exp_3 | 100 ± 36 | / | 221 ± 131 |
| **n=3** | | **141% ± 27** | **93% ± 30** |
| Statistiques: ****: p<0,0001 | | **** | **** |

Les extraits 1 et 1 bis de *Murraya koenigii* obtenus selon l'invention (bétaïne/propanediol/eau, ratio molaire 1:1:5) induisent une augmentation significative de la synthèse protéique de fibuline-5 déposée dans les fibroblastes dermiques. L'extrait 1 augmente (+141%) plus fortement que l'extrait 1bis (+93%) la synthèse protéique de fibuline-5 dans les fibroblastes dermiques.

Le solvant d'extraction seul (bétaïne/propanediol/eau ratio molaire 1:1:5 *sans Murraya koenigii*) n'induit pas d'augmentation de synthèse de fibuline-5 déposée (données non présentées).

### 6.4/ Conclusion

Les extraits 1 et 1bis (bétaïne/propanediol/eau, ratio molaire 1:1:5) de *Murraya koenigii* selon l'invention induisent une amélioration de la synthèse protéique du réseau élastique dans le derme.

### 7/ Exemple de composition - Gel crème naturel hydratant

**[Tableau 8]**

| Nom des ingrédients | Composition (% massique) |
|---|---|
| Emulium^{®} Mellifera | 2,5 |
| Squalane naturel | 2,0 |
| Dicaprylyl carbonate | 15,0 |
| Eau déminéralisée | QS100 |
| Glycérine | 20,0 |
| Amidon de tapioca | 5,0 |
| Extrait de *Murraya koenigii* selon l'invention | 2,0 |
| Ethylhéxylglycérine | 0,1 |
| Phénoxyéthanol | 0,9 |
| Parfum | 0,2 |
| Total | 100 |

## Revendications

1. Utilisation d'un extrait de *Murraya koenigii* étant obtenu par un procédé d'extraction solide/liquide, suivi d'une seconde étape de séparation solide/liquide et enfin d'une troisième étape de récupération de la phase liquide, **caractérisée en ce que** le solvant consiste en un mélange de bétaïne, de propanediol et d'eau, dans lequel les proportions molaires bétaïne:propanediol sont de 1: strictement inférieur à 1,5 ; ou d'une composition comprenant ledit extrait pour lutter contre le vieillissement cutané.

2. Utilisation selon la revendication 1, pour stimuler le processus d'élastogenèse cutané.

3. Utilisation selon l'une des revendications 1 ou 2, pour stimuler la formation de fibres élastiques cutanées.

4. Utilisation selon l'une des revendications 1 à 3, pour densifier la matrice extracellulaire cutanée.

5. Utilisation selon l'une des revendications 1 à 4, pour lutter contre le relâchement de la peau.

6. Utilisation selon l'une des revendications 1 à 5, **caractérisé en ce que** le solvant consiste en un mélange de bétaïne, de propanediol et d'eau, dans lequel les proportions molaires bétaïne:propanediol sont de 1:1.

7. Utilisation selon l'une des revendications 1 à 6, **caractérisée en ce que** l'eau représente entre 15 et 35% en poids du solvant.

8. Utilisation selon l'une des revendications 6 ou 7, **caractérisée en ce que** le solvant consiste en un mélange de bétaïne, de propanediol et d'eau dans des proportions molaires 1:1:5.

9. Extrait de *Murraya koenigii* étant obtenu par un procédé d'extraction solide/liquide, suivi d'une seconde étape de séparation solide/liquide et enfin d'une troisième étape de récupération de la phase liquide, **caractérisée en ce que** le solvant consiste en un mélange de bétaïne, de propanediol et d'eau, avantageusement dans des proportions molaires 1:1:5.

10. Extrait selon la revendication 9, **caractérisé en ce qu'**il s'agit d'un extrait de tiges foliaires et/ou de feuilles avantageusement séchées.

11. Extrait selon l'une des revendications 9 ou 10, **caractérisé en ce qu'**il est dépourvu d'alcaloïdes.

12. Extrait selon l'une des revendications 9 à 11, **caractérisé en ce qu'**il présente un rapport massique de tiges foliaires et/ou de feuilles de *Murraya koenigii*/solvant compris entre 1:99 et 50:50.

13. Composition cosmétique comprenant l'extrait selon l'une des revendications 9 à 12.

## Patentansprüche

1. Verwendung eines Extrakts von *Murraya koenigii,* der durch ein Fest/Flüssig-Extraktionsverfahren erhalten wird, gefolgt von einem zweiten Schritt der Fest/Flüssig-Trennung und schließlich einem dritten Schritt der Gewinnung der flüssigen Phase, **dadurch gekennzeichnet, dass** das Lösungsmittel aus einer Mischung aus Betain, Propandiol und Wasser besteht, wobei die molaren Anteile von Betain:Propandiol 1:streng unter 1,5 liegen; oder aus einer Zusammensetzung, die den Extrakt zur Bekämpfung der Hautalterung enthält.

2. Verwendung nach Anspruch 1, um den Elastogeneseprozess der Haut zu stimulieren.

3. Verwendung nach einem der Ansprüche 1 oder 2, um die Bildung von elastischen Hautfasern zu stimulieren.

4. Verwendung nach einem der Ansprüche 1 bis 3 zur Verdichtung der extrazellulären Hautmatrix.

5. Verwendung nach einem der Ansprüche 1 bis 4 zur Bekämpfung der Hauterschlaffung.

6. Verwendung nach einem der Ansprüche 1 bis 5, **dadurch gekennzeichnet, dass** das Lösungsmittel aus einer Mischung aus Betain, Propandiol und Wasser besteht, wobei die molaren Verhältnisse Betain:Propandiol 1:1 betragen.

7. Verwendung nach einem der Ansprüche 1 bis 6, **dadurch gekennzeichnet, dass** das Wasser zwischen 15 und 35 Gew.-% des Lösungsmittels beträgt.

8. Verwendung nach einem der Ansprüche 6 oder 7, **dadurch gekennzeichnet, dass** das Lösungsmittel aus einer Mischung aus Betain, Propandiol und Wasser in molaren Anteilen von 1:1:5 besteht.

9. Extrakt von *Murraya koeniggii,* der durch ein Verfahren der Fest/Flüssig-Extraktion erhalten wird, gefolgt von einem zweiten Schritt der Fest/Flüssig-Trennung und schließlich einem dritten Schritt der Gewinnung der flüssigen Phase, **dadurch gekennzeichnet, dass** das Lösungsmittel aus einer Mischung aus Betain, Propandiol und Wasser besteht, vorzugsweise in molaren Anteilen von 1:1:5.

10. Extrakt nach Anspruch 9, **dadurch gekennzeichnet, dass** es sich um einen Extrakt aus Blattstängeln und/oder Blättern handelt, die vorzugsweise getrocknet sind.

11. Extrakt nach einem der Ansprüche 9 oder 10, **dadurch gekennzeichnet, dass** er frei von Alkaloiden ist.

12. Extrakt nach einem der Ansprüche 9 bis 11, **dadurch gekennzeichnet, dass** er ein Gewichtsverhältnis von Blattstängeln und/oder Blättern von *Murraya koenigii*/Lösungsmittel zwischen 1:99 und 50:50 aufweist:

13. Kosmetische Zusammensetzung, die den Extrakt nach einem der Ansprüche 9 bis 12 enthält.

## Claims

1. Use of an extract of *Murraya koenigii* being obtained by a solid/liquid extraction process, followed by a second solid/liquid separation step and finally by a third step to recover the liquid phase, **characterized in that** the solvent consists of a mixture of betaine, propanediol and water, in which the betaine:propanediol molar proportions are 1: strictly less than 1.5; or of a composition comprising said extract to combat skin aging.

2. Use according to claim 1, for stimulating the process of skin elastogenesis.

3. Use according to one of claims 1 or 2, for stimulating the formation of skin elastic fibers.

4. Use according to one of claims 1 to 3, for densifying the cutaneous extracellular matrix.

5. Use according to one of claims 1 to 4, to combat skin slackening.

6. Use according to any one of claims 1 to 5, **characterized in that** the solvent consists of a mixture of betaine, propanediol and water, wherein the molar proportions of betaine:propanediol are 1:1.

7. Use according to any of claims 1 to 6, **characterized in that** the water represents between 15 and 35% by weight of the solvent.

8. Use according to one of claims 6 or 7, **characterized in that** the solvent consists of a mixture of betaine, propanediol and water in molar proportions 1:1:5.

9. Extract of *Murraya koenigii* being obtained by a solid/liquid extraction process, followed by a second solid/liquid separation step and finally by a third liquid phase recovery step, **characterized in that** the solvent consists of a mixture of betaine, propanediol, and water, advantageously in molar proportions of 1:1:5.

10. Extract according to claim 9, **characterized in that** it is an extract of leaf stalks and/or leaves advantageously dried.

11. Extract according to any of claims 9 or 10, **characterized in that** it is free of alkaloids.

12. Extract according to one of claims 9 to 11, **characterized in that** it has a mass ratio of leaf stems and/or leaves of *Murraya koenigii*/solvent of between 1:99 and 50:50.

13. Cosmetic composition comprising the extract according to any of claims 9 to 12.
